# EUROPEAN PATENT SPECIFICATION

(11) **EP 2 694 005 B1**
(45) Date of publication and mention of the grant of the patent: **16.12.2020**
(21) Application number: 12715771.7
(22) Date of filing: 28.03.2012
(51) Int. Cl.: A61F 13/02

(54) **NEGATIVE PRESSURE WOUND THERAPY DRESSING**
UNTERDRUCKWUNDBEHANDLUNGSAUFLAGE
SYSTÈME DE TRAITEMENT DES PLAIES PAR PRESSION NÉGATIVE

(30) Priority: 04.04.2011 US 201113079298
(43) Date of publication of application: 12.02.2014
(73) Proprietor: Smith & Nephew, Inc., Memphis, TN 38116 (US)
(72) Inventor: CORLEY, Kevin, Reading, MA 01867 (US)
(74) Representative: Smith & Nephew
(86) International application number: PCT/US2012/030829
(87) International publication number: WO 2012/138514

(56) References cited:
- EP-A2- 2 119 460
- GB-A- 2 415 908
- US-A1- 2007 027 414
- US-A1- 2007 129 707
- US-A1- 2007 161 938
- US-A1- 2008 009 812
- US-A1- 2010 286 635

## Description

### BACKGROUND

### 1. Technical Field

The present disclosure relates generally to wound dressings for use in a negative pressure wound therapy (NPWT) system. In particular, the disclosure relates to a composite wound dressing that includes a wound filler or packing member affixed to a cover layer to facilitate wound healing and ease the application of the dressing to a wound.

### 2. Background of Related Art

The body's natural wound healing process is a complex series of events beginning at the moment of injury. Initially the body reacts by delivering proteins and other factors to the wound through the blood stream to minimize the damage. Blood clots to prevent blood loss while cells engulf bacteria and debris to carry it away from the wound site. Next, the body begins to repair itself in a stage of healing often referred to as the proliferate phase. This phase is characterized by the deposition of granulation tissue in the wound bed. Granulation tissue provides a base structure over which cells may migrate inwardly from the periphery to close the wound. Finally the process ends as collagen gives strength to new tissue over time often forming a scar.

One technique for promoting the natural healing process, particularly, but not exclusively during the proliferate phase, is known as negative pressure wound therapy (NPWT). Application of a reduced pressure, e.g. sub-atmospheric, to a localized reservoir over a wound has been found to assist in closing the wound. The reduced pressure may be effective to promote blood flow to the area, to stimulate the formation of granulation tissue and the migration of healthy tissue over the wound by the natural process. Also a reduced pressure may inhibit bacterial growth by assisting in removing fluids exuding from the wound. This technique has proven effective for chronic or non-healing wounds, but has also been used for other purposes such as post-operative wound care.

The general NPWT protocol provides for the introduction of a filler or packing member into the wound, and subsequently applying a cover layer over wound and packing member. The packing member serves to support the cover layer and also to absorb and/or promote fluid transport away from the wound bed. The wound filler may comprise such materials as non-reticulated foams, non-woven fabrics, continuous fibers or gauze. The cover layer may comprise a thin polymeric film that includes an adhesive periphery for forming a substantially fluid tight seal with the healthy skin surrounding the wound. The cover layer thus defines a vacuum reservoir over the wound where a reduced pressure may be maintained over time by the application of individual or cyclic evacuation procedures.

In some instances, applying a packing member and a cover layer individually to a wound may be a time consuming, and labor intensive process. A composite wound dressing that includes a packing member affixed to a cover layer may facilitate the application of the wound dressing to a wound for use in a (NPWT) system.

US 2007/0027414 describes a laminated negative pressure wound dressing system.

US 2008/0009812 discloses an air-tight wound covering which when placed in contact with the body of the patient, forms a wound space between the respective wound and the wound cover.

GB 2415908 details a wound dressing for vacuum therapy where a screen structure is placed between the cover and the wound.

US 2010/0286635 is directed to a wound dressing preferably having a hydrophobic or biodegradable base layer and one or more absorptive layers for absorbing fluid from the wound.

### SUMMARY

The invention is as claimed in the claims.

The present disclosure describes a wound dressing suitable for use in negative pressure wound therapy system. The dressing includes a cover layer adapted to establish a reservoir over a wound in which a negative pressure may be maintained. The cover layer includes an opening therein through which atmospheric gasses and wound exudates may pass through the cover layer. A vacuum port is affixed to the cover layer, and establishes a substantially fluid tight seal about the opening in the cover layer. The vacuum port includes a hollow interior in fluid communication with the opening, and a connector to facilitate connection to a vacuum source. A packing member is affixed to the cover layer. The packing member is adapted to fill the wound and to support the cover layer when a negative pressure is applied to the reservoir.

The packing member includes a plurality of discrete elongated fingers extending distally from a distal side of the cover layer. The elongated fingers are constructed of strips of a foam material. The packing member may also include a supporting media affixed to the cover layer that defines a plurality of apertures therein through which a proximal end of the elongated fingers are interlaced. The supporting media may be constructed as a generally planar polymeric mesh. The supporting media may also define a flange of the vacuum port, or the supporting media may include a relatively pliable portion of a flange of the vacuum port.

The packing member may include a cellulose solution that is adapted to maintain the packing member in a relatively rigid condition prior to installation of the wound dressing, and a relatively pliable condition when the packing member is in contact with a wound. The cellulose solution may be arranged on an outer surface of the elongated fingers to substantially encapsulate the elongated fingers, allowing the clinician to strategically place the wound filler material in the wound bed without interfering with the application of the adhesive layer. The cellulose solution may be infused with vitamins and minerals that promote healthy wound healing, such as vitamin E.

According to another aspect of the disclosure, a wound dressing includes a cover layer adapted to establish a reservoir over a wound in which a negative pressure may be maintained. The cover layer includes an opening therein through which atmospheric gasses and wound exudates may pass through the cover layer. A vacuum port is affixed to the cover layer, and establishes a substantially fluid tight seal about the opening in the cover layer. The vacuum port includes a hollow interior in fluid communication with the opening, and a connector to facilitate connection to a vacuum source. A packing member is permanently affixed to a distal side of the cover layer, and includes a plurality of discrete elongated fingers extending distally from the distal side of the cover layer.

The cover layer may include an adhesive coating on the distal side thereof, and an adhesive bond may be established between the cover layer and the packing member by the adhesive coating. A release sheet or liner may be affixed to the cover layer by the adhesive coating on the distal side of the cover layer such that the packing member is interposed between the release sheet and the cover layer. The packing member may include a polymeric foam material cut such that each of the elongated fingers extends from continuous portion of the foam material. In some embodiments, the elongated fingers of the packing member may extend through an opening in the release liner.

According to another aspect of the present disclosure, a system for negative or subatmospheric pressure therapy in connection with healing a wound includes a cover layer dimensioned for positioning relative to a wound bed of a subject to establish a reservoir over the wound bed in which a negative pressure may be maintained. A packing member is permanently affixed to a distal side of the cover layer, and includes a polymeric foam material. The system also includes a vacuum source in fluid communication with the wound bed.

### BRIEF DESCRIPTION OF THE DRAWINGS

The accompanying drawings, which are incorporated in and constitute a part of this specification, illustrate embodiments of the present disclosure and, together with the detailed description of the embodiments given below, serve to explain the principles of the disclosure The invention is defined in the appended claims. Any disclosure lying outside the scope of said claims is only intended for illustrative as well as comparative purposes.
**FIG. 1** is a cross-sectional view of a negative pressure wound therapy apparatus with a wound dressing installed over a wound in accordance with the present disclosure;
**FIG. 2** is a cross-sectional view of the wound dressing of **FIG. 1** as packaged prior to application over the wound;
**FIG. 3** is a distal or wound-facing side view of the wound dressing of **FIG. 1** depicting a spiral die-cut packing member affixed to a cover layer;
**FIG. 4** is a cross-sectional view of an alternate embodiment of a wound dressing depicting a packing member cut to form elongated fingers;
**FIG. 5** is a cross-sectional view of an another alternate embodiment of a wound dressing the wound dressing including elongated fingers encapsulated in a thin shell;
**FIG. 6** is an exploded, perspective view of an alternate embodiment of a wound dressing including a packing member woven into a supporting media; and
**FIG. 7** is a partial, perspective view of an alternate embodiment of a wound dressing including a port member including a flange serving as a supporting media for a packing member.

### DETAILED DESCRIPTION OF PREFERRED EMBODIMENTS

The attached figures illustrate exemplary embodiments of the present disclosure and are referenced to describe the embodiments depicted therein. Hereinafter, the disclosure will be described in detail by explaining the figures wherein like reference numerals represent like parts throughout the several views.

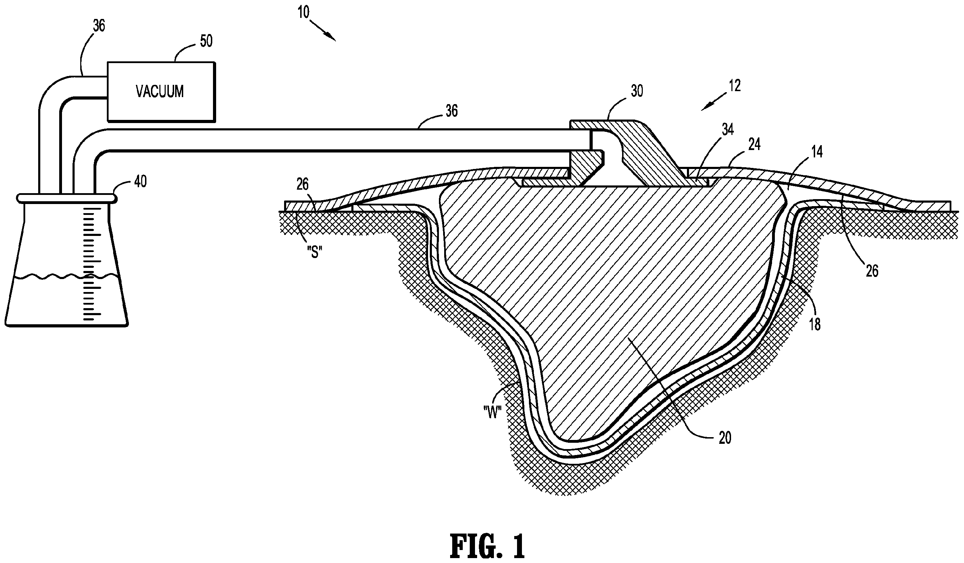

Referring initially to **FIG. 1**, an apparatus **10** for negative pressure wound therapy (NPWT) is depicted for use on a wound **"w"** surrounded by healthy skin **"s"** in accordance with the present disclosure. Apparatus **10** may be used to subject the wound **"w"** to a negative pressure continuously, or in varying intervals depending on the nature and severity of the wound **"w."** The use of a wound dressing in this manner has been found to promote healing by reducing the probability of infection, stimulating the deposition of granulation tissue and other beneficial processes. To facilitate the application of a negative pressure, the apparatus **10** includes a wound dressing **12** positioned relative to the wound **"w"** to define a reservoir **14** in which a negative pressure appropriate to stimulate healing may be maintained.

Wound dressing **12** includes an optional contact layer **18** positioned in direct contact with the bed of wound **"w."** Contact layer **18** may comprise a porous film including apertures therein permitting the negative pressure applied to the reservoir **14** to penetrate into the wound **"w,"** and also permitting exudates to be drawn from the wound **"w."** Three-dimensional formed and apertured films such as those provided by Tredegar Film Products of Richmond, VA, may be suitable for constructing contact layer **18.**

Packing member **20** is positioned in the wound **"w"** over the contact layer **18** and is intended to allow wound dressing **12** to absorb and capture wound exudates, or to transport wound exudates away from the wound **"w"** and out of the dressing **12.** Packing member **20** is shaped to be conformable to the shape of wound **"w,"** and to slightly overfill the wound **"w."** Alternatively, the packing member **20** may be sized and shaped to fill the wound "w" up to the level of healthy skin **"s."**

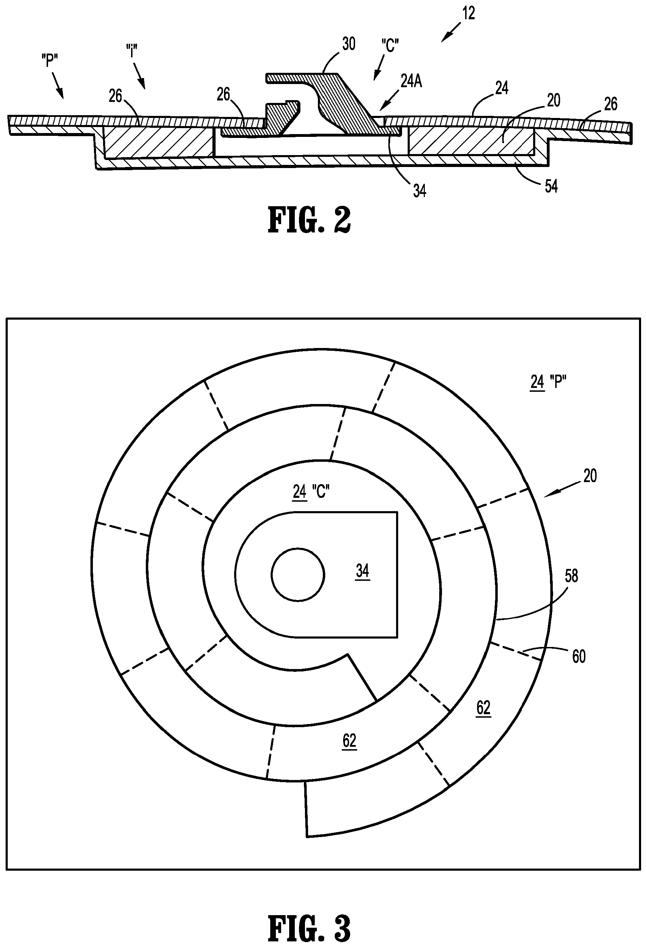

The packing member **20** may be constructed of an absorbent material such as gauze, reticulated foam, continuous fibers or alginate fibers to receive or transport any exudate that migrates through the contact layer **18.** One particular antimicrobial dressing, commercially available under the trademark Covidien™ AMD offered by Tyco Healthcare Group LP (d/b/a Covidien), may be suitable for use as packing member **20.** The Covidien™ AMD Foam dressing is polyurethane-based foam including the antiseptic agent polyhexamethylene biguanide (PHMB). A microstructured open-celled surface on the foam pad promotes absorption of exudates, and the added PHMB attacks bacteria on and within the dressing **12.** The Covidien™ AMD Foam dressing is described in greater detailed below with reference to **FIG. 2****.**

To discourage adhesion to the wound **"w,"** the packing member **20** may also comprise a material configured such that any stray fibers do not tend to protrude through apertures formed in contact layer **18** where they may become engulfed by newly forming granulation tissue. One particular type of material exhibiting this characteristic is formed of a tow of continuous filaments comprising either natural or man-made fibers. Continuous filaments include those relatively long strands of a synthetic material such as nylon, rayon, etc., which may offer a smooth continuous outer surface substantially free of the protruding fibrils commonly associated with natural materials such as cotton. The use of continuous filaments of a hydrophobic material such as polyolefin may permit a complete removal of packing member **20** when the dressing **12** is changed without re-injuring the wound **"w."**

Wound dressing **12** also includes a cover layer **24.** Cover layer **24** is positioned over the wound **"w"** such that an adhesive **26** on an underside of the cover layer **24** forms a substantially fluid-tight seal with the surrounding skin **"s."** Thus, cover layer **24** may act as both a microbial barrier to prevent contaminants from entering the wound **"w,"** and also a fluid barrier to maintain the integrity of vacuum reservoir **14.** The adhesive **26** on the underside of the cover layer **24** also affixes the packing member **20** to the cover layer **24.** Thus, the cover layer **24** and the packing member **20** may be installed on over the wound **"w"** together as a single component.

Cover layer **24** is preferably formed from a moisture vapor permeable membrane to promote the exchange of oxygen and moisture between the wound "**w**" and the atmosphere, and is preferably transparent permit a visual assessment of wound conditions without requiring removal of the cover layer **24.** A transparent polyurethane membrane providing a sufficient moisture vapor transmission rate (MVTR) for use as cover layer **24** is sold under the trade name POLYSKIN®II by Tyco Healthcare Group LP (d/b/a Covidien). Alternatively, cover layer **24** may comprise an impermeable membrane or a substantially rigid member.

An optional vacuum port **30** having a flange **34** may also be included in wound dressing **12** to facilitate connection of the wound dressing **12** to a fluid conduit **36.** An upper surface of the flange **34** is adhered to the cover layer **24** by the adhesive **26** on the underside of the cover layer **24.** Thus, the vacuum port **30** is affixed to the cover layer **24,** and may be installed over the wound **"w"** together with the cover layer **24** and the packing member **20** as a single component. Alternatively, an adhesive (not shown) on the underside the flange **34** may be provided such that the vacuum port **30** may be installed over the cover layer **24.** Vacuum port **30** may be provided as a pre-affixed component of dressing **12,** as a component of fluid conduit **36** or entirely independently. Alternatively, vacuum port **30** may be eliminated from dressing **12** if other provisions are made for providing fluid communication with the fluid conduit **36.**

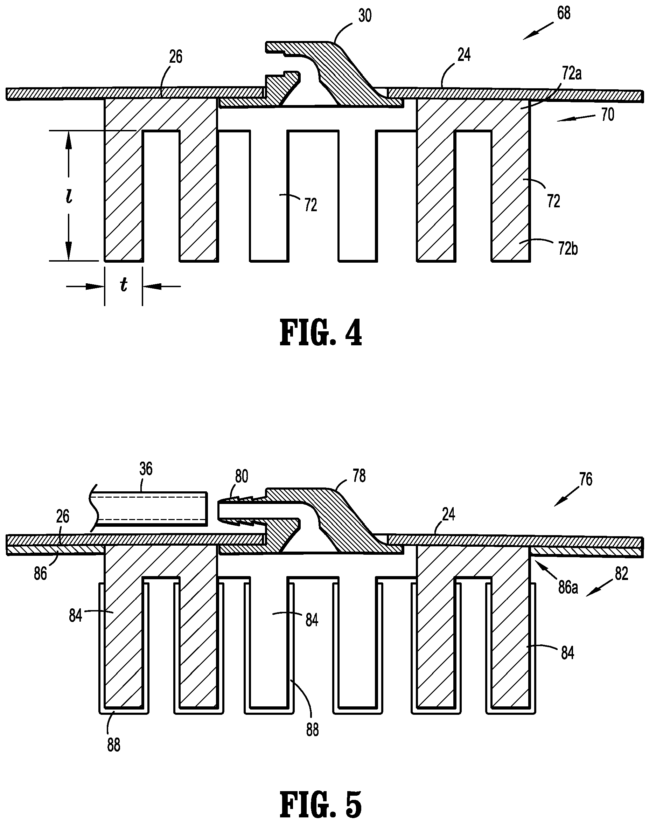

The vacuum port **30** may be constructed of a polypropylene material and may be configured as a rigid or flexible, low-profile component. The vacuum port **30** may be adapted to receive fluid conduit **36** in a releasable and fluid-tight manner. An outer surface of the fluid conduit **36** may frictionally engage an inner surface of the vacuum port **30.** Alternatively, a barbed connector (see **FIG. 5**), quick disconnect, bayonet coupling or permanent coupling methods may be employed to couple the fluid conduit to the vacuum port **30.** A hollow interior of the vacuum port **30** provides fluid communication between the fluid conduit **36** and the reservoir **14.**

Fluid conduit **36** extends from the vacuum port **30** to provide fluid communication between the reservoir **14** and a fluid collection canister **40.** Any suitable conduit may be used for fluid conduit **36** including those fabricated from flexible elastomeric or polymeric materials. Fluid conduit **36** may connect to the canister **40** in a fluid-tight manner by any appropriate mechanism including any of the mechanisms described above for connecting the fluid conduit to the vacuum port **30.**

Collection canister **40** may comprise any container suitable for containing wound fluids. For example, a rigid bottle may be used as shown or alternatively a flexible polymeric pouch may be appropriate. Collection canister **40** may contain an absorbent material to consolidate or contain the wound drainage or debris. For example, super absorbent polymers (SAP), silica gel, sodium polyacrylate, potassium polyacrylamide or related compounds may be provided within canister **40.** At least a portion of canister **40** may be transparent to assist in evaluating the color, quality or quantity of wound exudates. A transparent canister may thus assist in determining the remaining capacity of the canister or when the canister should be replaced.

Leading from collection canister **40** is another section of fluid conduit **36** providing fluid communication with vacuum source **50.** Vacuum source **50** generates or otherwise provides a negative pressure to the NWPT apparatus **10.** Vacuum source **50** may comprise a peristaltic pump, a diaphragmatic pump or other mechanism that is biocompatible and draws fluids, e.g. atmospheric gasses and wound exudates, from the reservoir **14** appropriate to stimulate healing of the wound **"w."** Preferably, the vacuum source **50** is adapted to produce a sub-atmospheric pressure in the reservoir **14** ranging between about 20 mmHg and about 500 mmHg, more preferably, about 75 mmHg to about 125 mmHg, or more preferably, about 40 mmHg to about 80 mmHg.

Referring now to **FIG. 2****,** a wound dressing **12** is depicted in an initial configuration prior to application over the wound **"w"** (**FIG. 1**). The packing member **20,** cover layer **24** and the vacuum port **30** are all coupled to one another by the adhesive **26** on the underside of the cover layer **24.** The vacuum port **30** extends through a central opening **24a** in the cover layer **24** and is adhered to a radially central region **"c"** of the cover layer **24** by the flange **34.** The flange **34** establishes a substantially fluid tight seal with the cover layer **24.** The packing member **20** radially surrounds the vacuum port **30** and is adhered to a radially intermediate region **"i** " of the cover layer **24.** A release liner **54** is adhered to the adhesive **26** at a peripheral region **"p"** of the cover layer **24** and serves to protect the adhesive **26** and the packing member **20** prior to use of the dressing **12.** The release liner **54** may be constructed of a silicone coated paper or similar material that will readily detach from the cover layer **26** without inhibiting ability of the adhesive **26** in the peripheral region "p" to form a fluid-tight seal with the skin **"s"** (**FIG. 1**).

The packing member **20** depicted in **FIG. 2** is constructed as a die-cut pad or disc of the Covidien™ AMD foam, and is adhered to the cover layer **24** by the adhesive **26** radially surrounding the vacuum port **30.** The packing member **20** assumes a generally planar cross-section and occupies a relatively small volume prior to exposure to a moist wound environment. The planar configuration of the packing member **20** is suitable for use in wounds such as shallow ulcers that generally do not require a large amount of material to fill the wound. The tendency of the Covidien™ AMD foam to swell in the presence of moisture helps fill the void of the wound "w" (**FIG. 1**), and thus, provides support to the cover layer **24** as the wound **"w"** is subjected to negative pressure. The tendency to swell in the presence of moisture also helps to maintain intimate contact between the packing member **20** and the wound bed "w" (**FIG. 1**). The continuous nature of a die-cut foam pad alleviates a risk of portions of the packing member being inadvertently left in the wound **"w"** when the wound dressing **12** is removed. The microstructured open celled surface on the foam pad discourages ingrowth of tissue from a wound site into the matrix of the packing member **20,** and thus alleviates the risk of reinjuring a wound when the wound dressing **12** is removed or changed. In other embodiments (not shown) a gauze pad or non-woven wound dressing sponge may be affixed to the distal side of the foam packing member **20.**

Referring now to **FIG. 3****,** the packing member **20** is die-cut to assume a spiral configuration. A spirally shaped major seam **56** is cut between successive windings or layers of the spiral, and a plurality of minor seams **60** is cut laterally into the windings of the spiral to define a plurality of discrete components **62.** The seams **56, 60** may extend entirely through the packing member **20** to define the discrete separable components **62** of the spiral. Alternately, the seams **56, 60** may extend partially through the packing member **20** or may be defined by a series of perforations to permit each of the discrete components **62** to be readily separable from the spiral. Thus, a clinician may unwind the spiral and tear or cut any number of discrete components **62** from the spiral as necessary to conform the packing member **20** to the size of the wound "**w**."

Referring now to **FIG. 4****,** an alternate embodiment of a wound dressing **68** includes a cover layer **24** and a vacuum port **30** affixed thereto in a manner substantially similar to the wound dressing **12** described above with reference to **FIG. 2****.** The wound dressing **68** also includes a packing member **70** affixed to the distal side of the cover layer **24.** The packing member **70** includes a plurality of elongated fingers **72** that may be cut to an appropriate size or length at the time the wound dressing **68** is installed to accommodate a particular wound configuration. The packing member **70** may be constructed of any of the materials discussed above for construction of the packing member **20** (**FIG. 2**) including a tow of continuous filaments, and a die-cut open celled foam having fingers **72** cut therein.

Each of the elongated fingers includes a proximal end **72a** affixed to the cover layer **24,** and a free end **72b** extending therefrom in a distal direction. Where the packing member **70** is constructed of a foam material, the elongated fingers **72** may have a length "**ℓ**" in the range of about 1 inch to about 4 inches, and a thickness **"** 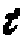 " in the range of about 0.125 inches to about 0.5 inches. In other embodiments, where the packing member **70** is constructed of a tow of continuous filaments, the elongated fingers **72** may be substantially longer and narrower. The fingers **72** are supported by the adhesive **26** on the distal side of the cover layer, and may hang down from the cover layer **24** into the wound **"w"** when as the dressing **12** is installed.

Referring now to **FIG. 5****,** an alternate embodiment of a wound dressing **76** includes a cover layer **24** and a vacuum port **78** affixed thereto. The vacuum port **78** includes a barbed connector **80** for engaging an interior surface of a tubing section **36** in a fluid-tight manner. In other embodiments (not shown) the tubing section **36** and vacuum port **78** may the provided or fitted with a universal connector. A universal connector is a connector of a type used to interchangeably connect a variety of different components to one another, as opposed to a propriety connector used to connect only a specific component to another specific component. A universal connector may, for example, employ an industry standard connector type, such that the dressing **76** may be used in conjunction with a wide variety of standard medical equipment.

The wound dressing **76** includes a packing member **82** affixed to the distal side of the cover layer **24,** and the packing member **82** includes a plurality of elongated fingers **84** extending in a distal direction from the cover layer **24** in a manner substantially similar to the elongated fingers **72** described above with reference to **FIG. 4****.** The elongated fingers **84** could also be constructed of a continuous tow treated with PHMB for microbial efficacy. The wound dressing **76** includes a release sheet or liner **86** adhered to the adhesive **26** of the cover layer **24,** and the release liner **86** includes an opening **86a** therein through which the packing member **82** and the elongated fingers **84** extend. The opening **86a** in the release liner **86** permits a substantially flat release liner **86** to protect the adhesive **26** prior to use of the dressing **76,** and may facilitate application of the release liner **86** to the cover layer **24** during manufacturing of the dressing **76.**

The elongated fingers **84** are each encapsulated in a thin shell **88** disposed on an exterior surface of the respective finger **84.** The shell **88** is constructed of a cellulose solution that may contain proteins and vitamins that could aid in the wound healing process. The cellulose serves as a stiffening agent that provides some rigidity to the fingers **84** to facilitate handling and placement of the wound dressing **76.** Once the fingers are wetted by the moist wound environment, the fingers **84** will transition to a soft and pliable configuration that promotes patient comfort. In other configurations, the shell **88** could be constructed of a polymeric material having a glass transition temperature above room temperature (i.e., above about 25°C), but below body temperature (i.e., below about 37°C). At room temperature, the coating stiffens the fingers **84** for ease in handling and manipulation, and upon placement in a wound **"w,"** the shell **88** will soften as the temperature rises above the glass transition temperature of the polymeric material, thereby rendering the fingers pliable so that they conform to the contours of the wound **"w."** In still other configurations, the fingers **84** may be soaked in the cellulose solution or polymeric material such that the stiffening agent is absorbed into the matrix of the fingers **84** rather than being disposed on an exterior surface as shell or coating.

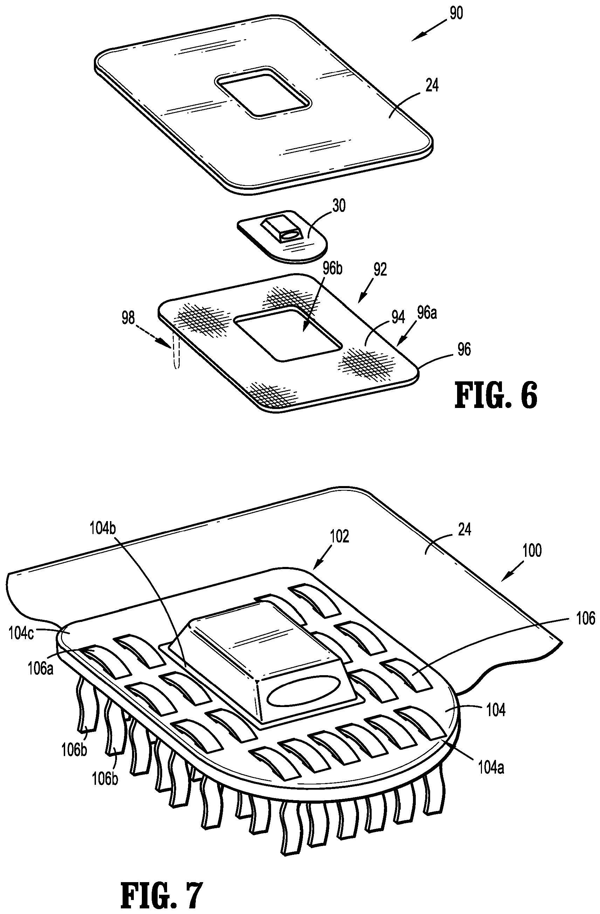

Referring now to **FIG. 6****,** an alternate embodiment of a wound dressing **90** includes a cover layer **24** and a vacuum port **30** substantially similar to the cover layer **24** and vacuum port **30** of wound dressing **12** described above with reference to **FIG. 2****.** The wound dressing **90** also includes a packing member **92** that may be affixed to the distal side of cover layer **24** such that the entire wound dressing **90** may be applied over the wound **"w"** (**FIG. 1**) as a single component. The packing member **92** is constructed of a continuous tow **94** woven into a supporting media **96.** The supporting media **96** is constructed as a generally planar, polypropylene screen or mesh including apertures **96a** therein that are sized and shaped to accommodate the tow **94** interlaced therethrough. A central opening **96b** is defined through the supporting media **96** that is sized to accommodate the vacuum port **30.** The packing member **92** may thus be affixed to the cover layer **24** to radially surround the vacuum port **30.** The tow **94** may be woven closely to the supporting media **96** such that the packing member **92** defines a substantially flat pad, or alternatively, the tow **94** may be arranged to protrude or hang in a distal direction from the supporting media **96** such that the tow **94** defines elongated fingers **98** as depicted in phantom. In other embodiments (not shown) the supporting media **96** may be configured to receive thin strips of a foam material, woven medical fabrics, or non-woven material woven therethrough.

Referring now to **FIG. 7****,** an alternate embodiment of a wound dressing **100** includes a cover layer **24** and a vacuum port **102** affixed thereto. The vacuum port **102** includes a flange **104** that serves to affix the vacuum port **102** to the cover layer **24** in a substantially fluid-tight manner, and also serves to provide a supporting media for thin strips of a foam material **106.** The strips **106** include a proximal end **106a** interlaced through apertures **104a** defined in the flange **104,** and a pair of free ends **106b** extending from the flange **104** in a distal direction. The free ends **106b** may be cut to an appropriate length at the time of application to a wound **"w"** (**FIG. 1**).

In some embodiments, a central portion **104b** of the flange **104** may be constructed to exhibit a relative rigidity with respect to a radial extension **104c** of the flange **104,** which is constructed to exhibit a relatively pliability. The relatively pliable extension **104c** may flex and bend with the cover layer **24** during evacuation cycles and promote patient comfort. The extension **104c** may be constructed of a more pliable material than the central portion **104b,** or alternatively, the extension **104c** may be constructed with a tapering or reduced thickness with respect to the central portion **104b.**

## Claims

1. A wound dressing (10), which comprises:
a cover layer (24) adapted to establish a reservoir (14) over a wound in which a negative pressure may be maintained, the cover layer (24) including an opening therein through which atmospheric gasses and wound exudates may pass through the cover layer (24),
a vacuum port (78) affixed to the cover layer (24) to establish a substantially fluid tight seal about the opening in the cover layer (24), the vacuum port (78) including a hollow interior in fluid communication with the opening in the cover layer (24), and a connector to facilitate connection to a vacuum source; and
a packing member (70) affixed to the cover layer, the packing member (70) adapted to fill the wound and to support the cover layer (24) when a negative pressure is applied to the reservoir (14); and **characterised in that** the packing member includes a plurality of discrete elongated fingers extending distally from a distal side of the cover layer and wherein the elongated fingers (72) are constructed of strips of a foam material.

2. The wound dressing according to claim 1, wherein the packing member is arranged to radially surround the vacuum port.

3. The wound dressing (10) according to claim 1, wherein the packing member includes a supporting media affixed to the cover layer, the supporting media defining a plurality of apertures therein through which a proximal end of the elongated fingers is interlaced.

4. The wound dressing (10) according to claim 3, wherein the supporting media is constructed as a generally planar polymeric mesh.

5. The wound dressing (10) according to claims 3 or 4, wherein the supporting media (96) defines a flange (34) of the vacuum port (78).

6. The wound dressing (10) according to claim 1, wherein the packing member (82) includes a cellulose solution adapted to maintain the packing member in a relatively rigid condition prior installation of the wound dressing, and a relatively pliable condition when the packing member is in contact with a wound.

7. The wound dressing (10) according to claim 6, wherein the cellulose solution is arranged on an outer surface of the elongated fingers (72) to substantially encapsulate the elongated fingers (72).

## Patentansprüche

1. Ein Wundverband (10), der Folgendes beinhaltet:
eine Deckschicht (24), die angepasst ist, um ein Reservoir (14) über einer Wunde zu errichten, in dem ein Unterdruck gehalten werden kann, wobei die Deckschicht (24) eine Öffnung darin umfasst, durch die atmosphärische Gase und Wundexsudate durch die Deckschicht (24) hindurchgehen können,
einen Vakuumanschluss (78), der an der Deckschicht (24) angebracht ist, um eine im Wesentlichen fluiddichte Abdichtung über der Öffnung in der Deckschicht (24) zu errichten, wobei der Vakuumanschluss (78) einen hohlen Innenraum, der in Fluidkommunikation mit der Öffnung in der Deckschicht (24) steht, und einen Verbinder, um die Verbindung mit einer Vakuumquelle zu erleichtern, umfasst; und
ein Packelement (70), das an der Deckschicht angebracht ist, wobei das Packelement (70) angepasst ist, um die Wunde zu füllen und die Deckschicht (24) zu unterstützen, wenn ein Unterdruck auf das Reservoir (14) angewendet wird; und **dadurch gekennzeichnet, dass** das Packelement eine Vielzahl von einzelnen länglichen Fingern umfasst, die sich von einer distalen Seite der Deckschicht distal erstrecken, und wobei die länglichen Finger (72) aus Streifen eines Schaumstoffs gefertigt sind.

2. Wundverband gemäß Anspruch 1, wobei das Packelement angeordnet ist, um den Vakuumanschluss radial zu umgeben.

3. Wundverband (10) gemäß Anspruch 1, wobei das Packelement ein Unterstützungsmedium umfasst, das an der Deckschicht angebracht ist, wobei das Unterstützungsmedium eine Vielzahl von Durchlässen darin definiert, durch die ein proximales Ende der länglichen Finger geflochten ist.

4. Wundverband (10) gemäß Anspruch 3, wobei das Unterstützungsmedium als ein im Allgemeinen ebenes polymeres Netz gefertigt ist.

5. Wundverband (10) gemäß Ansprüchen 3 oder 4, wobei das Unterstützungsmedium (96) einen Flansch (34) des Vakuumanschlusses (78) definiert.

6. Wundverband (10) gemäß Anspruch 1, wobei das Packelement (82) eine Celluloselösung umfasst, die angepasst ist, um das Packelement vor der Einrichtung des Wundverbands in einem relativ unbiegsamen Zustand und in einem relativ biegsamen Zustand, wenn das Packelement in Kontakt mit einer Wunde steht, zu halten.

7. Wundverband (10) gemäß Anspruch 6, wobei die Celluloselösung auf einer Außenfläche der länglichen Finger (72) angeordnet ist, um die länglichen Finger (72) im Wesentlichen einzukapseln.

## Revendications

1. Un pansement pour plaie (10), lequel comprend :
une couche de recouvrement (24) conçue pour établir un réservoir (14) par-dessus une plaie dans lequel une pression négative peut être maintenue, la couche de recouvrement (24) incluant une ouverture dans celle-ci à travers laquelle des gaz atmosphériques et des exsudats de plaie peuvent traverser la couche de recouvrement (24),
un orifice de vide (78) fixé à la couche de recouvrement (24) afin d'établir un joint substantiellement étanche à des fluides autour de l'ouverture dans la couche de recouvrement (24), l'orifice de vide (78) incluant un intérieur creux en communication fluidique avec l'ouverture dans la couche de recouvrement (24), et un connecteur afin de faciliter une connexion à une source de vide ; et
un élément de garniture (70) fixé à la couche de recouvrement, l'élément de garniture (70) étant conçu pour remplir la plaie et pour supporter la couche de recouvrement (24) lorsqu'une pression négative est appliquée sur le réservoir (14) ; et **caractérisé en ce que** l'élément de garniture inclut une pluralité de doigts allongés discrets s'étendant distalement depuis un côté distal de la couche de recouvrement et où les doigts allongés (72) sont construits de bandes d'un matériau en mousse.

2. Le pansement pour plaie selon la revendication 1, où l'élément de garniture est agencé pour entourer radialement l'orifice de vide.

3. Le pansement pour plaie (10) selon la revendication 1, où l'élément de garniture inclut un milieu de support fixé à la couche de recouvrement, le milieu de support définissant une pluralité de fenêtres dans celui-ci à travers lesquels une extrémité proximale des doigts allongés est entrelacée.

4. Le pansement pour plaie (10) selon la revendication 3, où le milieu de support est construit en forme de maille polymère généralement plane.

5. Le pansement pour plaie (10) selon les revendications 3 ou 4, où le milieu de support (96) définit une collerette (34) de l'orifice de vide (78).

6. Le pansement pour plaie (10) selon la revendication 1, où l'élément de garniture (82) inclut une solution de cellulose conçue pour maintenir l'élément de garniture dans un état relativement rigide préalablement à l'installation du pansement pour plaie, et un état relativement pliable lorsque l'élément de garniture est en contact avec une plaie.

7. Le pansement pour plaie (10) selon la revendication 6, où la solution de cellulose est agencée sur une surface externe des doigts allongés (72) afin d'encapsuler substantiellement les doigts allongés (72).
